# EUROPEAN PATENT SPECIFICATION

(11) **EP 3 344 313 B1**
(45) Date of publication and mention of the grant of the patent: **03.11.2021**
(21) Application number: 16840404.4
(22) Date of filing: 26.08.2016
(51) Int. Cl.: A61D 7/00, A61M 5/24, A61M 5/31, A61M 5/32, A61D 1/06, A61M 5/46, A61M 5/315

(54) **AN INJECTOR**
INJEKTOR
INJECTEUR

(30) Priority: 31.08.2015 AU 2015903539
(43) Date of publication of application: 11.07.2018
(73) Proprietor: 4C Design Limited, Glasgow, Strathclyde G4 9XG (GB); Meat & Livestock Australia Limited, North Sydney, New South Wales, 2059 (AU)
(72) Inventor: SMITH, Robin, Hornsby, New South Wales 2077 (AU); WILSON, Joe, Hornsby, New South Wales 2077 (AU); PENVER, Mark, Hornsby, New South Wales 2077 (AU); RICHIE, John, Hornsby, New South Wales 2077 (AU); HARRIS, Tom, Hornsby, New South Wales 2077 (AU)
(74) Representative: Mewburn Ellis LLP
(86) International application number: PCT/AU2016/050792
(87) International publication number: WO 2017/035575

(56) References cited:
- WO-A1-2012/045833
- WO-A1-2012/173554
- WO-A1-2015/011488
- WO-A2-2013/034984
- WO-A2-2013/034984
- GB-A- 2 451 666
- GB-A- 2 469 672
- GB-A- 2 482 241
- GB-A- 2 507 541
- US-A1- 2001 028 118
- US-A1- 2013 046 249
- US-A1- 2013 324 925
- US-A1- 2015 045 742

## Description

### FIELD OF THE INVENTION

The present invention relates to an injector. The invention finds particular, but by no means limiting, application for use with a castration tool to deliver an anaesthetic and/or analgesic to an animal body part to be castrated.

### BACKGROUND OF THE INVENTION

It is common practice to castrate male lambs at an early age for purposes including the prevention of undesired breeding and improved meat production. By far the most common method of doing so is by the application of a rubber ring to the scrotum above the testicles, which restricts the blood supply and leads to the atrophy and eventual dropping-off of the scrotum and testicles. A tool is used to apply the rubber ring, usually in the form of a pliers-like instrument having four posts which locate within and spread the ring for locating over the testicles.

This procedure causes some concern on animal welfare grounds. The behaviour of lambs during the procedure indicates that there is a degree of pain which typically lasts some hours.

V. Molony et al, "Pain in lambs castrated at 2 days using novel smaller and tighter rubber rings without and with local anesthetic", Veterinary Journal (London, England) 2012; 193(1):81-6, describes an experiment in which rubber rings of smaller than usual size and of a different shape were used. The hypothesis was that the application of a higher degree of compression would produce loss of sensation by direct compression of the nerves. However, this proved not to be the case, and this was attributed to the presence of scrotal skin folds and wool preventing suitable compression of the nerves being achieved.

This paper also describes the use of local anesthetic in one group of lambs in conjunction with the ring application. However, this was done as a separate operation using a needle-less injector. Such a procedure is unsuitable for commercial use where the operation is effected in very poor conditions in the field by farmers and shepherds. Moreover, the operator is normally working alone and has to restrain the lamb while carrying out the procedure; it is therefore desirable in practical terms to effect the castration procedure with one hand, leaving the other hand free for use in restraining the lamb

Injectors with two-stage injection mechanisms are known in the art, see, for example, document WO 2013/034984 A2.

### SUMMARY OF THE INVENTION

In accordance with a first aspect of the invention there is provided an injector having a two stage injection comprising:
an outer housing having a first end and a second end, the outer housing comprising a cylindrical hollow body having one or more helical cams disposed on an internal wall within the body at the first end;
a cylindrical hollow rotor body having one or more cam bearing portions disposed at one end and at least one guide slot disposed in a circumferential wall, each guide slot comprising a first transverse portion extending into a second longitudinal portion, the rotary body being concentrically located within the body of the outer housing such that each cam bearing portion faces a corresponding helical cam;
a plunger assembly comprising:
   a barrel for storing a fluid;
   a needle disposed at a first end of the barrel; and
   a plunger for dispensing the fluid through the needle; and wherein at least one guide projection is disposed on a circumferential wall of the plunger;
the plunger assembly being at least partially locatable within the rotor body such that each guide projection locates in a corresponding guide slot and such that when the projections are located in the transverse portion the plunger is constrained from movement toward the first end of the barrel and when located in the longitudinal portion the plunger is free to move toward the first end of the barrel;
in use the plunger assembly and rotor body being moveable within the outer housing between a retracted position wherein the needle is at least partially housed within the outer housing and an engaged position wherein the needle projects outwardly of a needle opening located at the first end of the outer housing,
characterized in that, the plunger assembly and rotor body are moveable to the engaged position responsive to a force aligned with the direction of movement of the plunger assembly being applied to a second end of the plunger assembly, and such that continued application of the force causes the one or more cam bearing portions to bear on the one or more helical cams, thereby causing the rotor body to rotate within the outer housing in turn causing the guide projections to align with longitudinal portion of the corresponding guide slot, thereby permitting movement of the plunger for dispensing the fluid.

The plunger assembly is moveable toward the engaged position responsive to a force being applied to a second end of the plunger assembly.

Once the plunger assembly reaches the engaged position, continued application of the force causes the non-inhibited plunger to move within the barrel for expelling stored contents through the needle.

The rotary inhibiting mechanism comprises a generally cylindrical hollow rotor body concentrically located within the outer housing and wherein at least a portion of the plunger assembly is located within the rotor body.

Once located within the rotor body, both the plunger assembly and rotor body are correspondingly movable within the outer housing between the retracted and engaged position.

In an embodiment the rotor body and plunger assembly are biased toward the retracted position by way of a return spring located within the outer housing.

In an embodiment the return spring surrounds and is co-axially aligned with the rotor body and wherein a portion of at least one of the rotor body and plunger assembly bears on a non-seated end of the spring.

The rotary inhibiting mechanism further comprises at least one guide slot defined in a circumferential wall of the rotor body for receiving a corresponding guide projection extending from the plunger assembly.

In an embodiment the barrel of the plunger assembly comprises a forward portion comprising a fluid storing chamber and an intermediate portion comprising a plunger which locates within the chamber.

In an embodiment the intermediate portion further comprises an outer wall which surrounds and receives the forward portion as the plunger is depressed.

In an embodiment the projections are lugs which extend outwardly from the outer wall of the intermediate portion.

In an embodiment each guide slot comprises a seating portion which is in perpendicular alignment to the movement of the plunger assembly within the outer body and which in turn extends into a longitudinally aligned stroke portion.

The outer housing further comprises one or more helical cams disposed on an internal wall, the cam(s) facing correspondingly located bearing portions disposed on a free end of the rotor body and wherein when the rotor body is moved toward the engaged position the helical cams contact the corresponding bearing portions thereby causing the rotor body to rotate within the outer housing.

In an embodiment, when in the retracted position, the rotor body is aligned within the outer housing such that each lug seats on a seating portion of the respective guide slot thereby inhibiting movement of the plunger.

In an embodiment as the rotor body is moved toward the engaging portion the rotor rotates causing the longitudinal stroke portions to be aligned with the respective lugs, such that once in the engaged position the lugs are free to travel along the stroke portions thereby permitting movement of the plunger for expelling the fluid.

In an embodiment the outer housing further comprises at least one longitudinally extending guide slot disposed in the circumferential wall configured to receive an end portion of a corresponding lug, wherein each end portion is held within and travels along the slot as the plunger assembly is operated thereby preventing rotational movement of the plunger assembly within the outer housing.

In an embodiment the injector further comprises a rotatable collar located at the second end of the outer housing and having locating channels disposed in an inner circumferential wall thereof.

In an embodiment the collar is rotatable between an open position whereby the locating channels are each axially aligned with and extend into respective guide slots in the outer housing for receiving a corresponding lug during insertion of the plunger mechanism into the outer housing and a closed position whereby the respective slots are no longer aligned, thereby preventing the plunger assembly from being removed from the outer housing.

In an embodiment the plunger assembly further comprising a fluid storage receptacle in fluid communication with the internal chamber of the barrel.

In an embodiment the fluid storage receptacle is a bottle coupled to the second end of the barrel.

In an embodiment the plunger assembly further comprises a fluid channel which, in use, extends through the plunger and is in fluid communication with an internal chamber of the receptacle.

In an embodiment the plunger assembly further comprises a non-return valve located in the fluid channel for providing a one way flow of fluid from the receptacle into the internal chamber.

In an embodiment the valve is a normally closed wire sprung check valve.

In an embodiment an opening of the bottle is fixedly coupled to the plunger by way of a collar.

In an embodiment prior to insertion of the plunger assembly into the outer housing, the opening of the bottle is sealed and set back from an opening in the fluid channel.

In an embodiment the plunger assembly further comprises a piercing portion disposed at an end of the plunger through which the opening in the fluid channel passes, during insertion of the plunger assembly into the outer housing a force applied to an end of the bottle causes the piercing portion to move toward the seal and broach the seal thereby allowing fluid to flow into the fluid channel.

In an embodiment the injector further comprises an air inlet comprising a one way valve located in the piercing portion for allowing air to enter the bottle once the seal has been breached.

In an embodiment the bottle is held in the breached position by way of a snap hook fitting.

In an embodiment the needle comprises a hypodermic needle.

In accordance with another aspect there is provided an apparatus for removal of an animal body part, the apparatus comprising: a ligature applicator configured to move a ligature between a first, extended state in which the ligature can be passed over the animal body part to a selected application position, and a second, retracted state in which the ligature applies a compressive force at the application position to restrict blood flow in the animal body part; and an injector as described above.

In an embodiment the injector is mounted to the ligature application such that the needle opening in the injector is in alignment with a portion of the ligature application which receives the animal body part and wherein operation of the injector causes the needle to move from a retracted position whereby the needle is remote from the body part to an engaged position whereby it is engaged with the body part for delivering the substance.

In an embodiment the substance is an analgesic or an anesthetic.

In an embodiment a first end of the outer housing of the injector is detachably received within an outer casing of the ligature applicator.

In an embodiment the ligature is a closed elastic ligature capable of being applied in a stretched condition.

In an embodiment the ligature is an elastic ring and wherein the ligature applicator may be configured to engage with and move the ligature from an initial, retracted state to the first, extended state.

In an embodiment the members of the ligature applicator are protruding pins moveable between a first position in which a ligature is held in an extended state and a second position in which the ligature is allowed to disengage from the members.

In an embodiment the apparatus is a hand tool comprising two handle portions, the two handle portions being pivotably connected to one another and configured such that movement of the two handle portions operates the ligature applicator to move the ligature members between the first and second positions.

### BRIEF DESCRIPTION OF THE ACCOMPANYING DRAWINGS

Embodiments of the present invention will now be described, by way of example only, with reference to the accompanying drawings, in which:
Figs. 1a and 1b show exploded perspective and side views respectively of an injector in accordance with an embodiment of the present invention;
Fig. 2 is a perspective view of the Fig. 1 injector in an assembled state;
Fig. 3 is a sectional side view of the assembled injector;
Fig. 4a shows an end, side and sectional view of the injector rotor sans the plunger mechanism;
Fig. 4b shows an end, side and sectional view of the rotor with plunger mechanism;
Fig. 5 shows various stages for locking the plunger assembly into the outer housing, in accordance with an embodiment;
Figs. 6a to 6c illustrate operation of the rotary inhibiting mechanism;
Figs. 7a and 7b are partial views of the plunger assembly in an assembly and in-use position, respectively;
Figs. 8a to 8d are side views of the injector in various stages of assembly;
Fig. 9 is a perspective view of a castration apparatus in accordance with an embodiment;
Fig. 10 shows the ligature applicator of the castration apparatus of Fig. 9 in use; and
Fig. 11 shows the injector of the castration apparatus of Fig. 9 in use.

### DETAILED DESCRIPTION OF EXEMPLARY EMBODIMENTS OF THE INVENTION

Embodiments of the invention will hereafter be described in the context of an injector having a two stage injection mechanism for delivery of an anaesthetic into an animal's tissue as during a castration procedure. It will be understood, however, that embodiments are not so limited and the injector could be used for delivery of any desired fluid, or combination of fluids, into any human or animal tissue for any desired purpose.

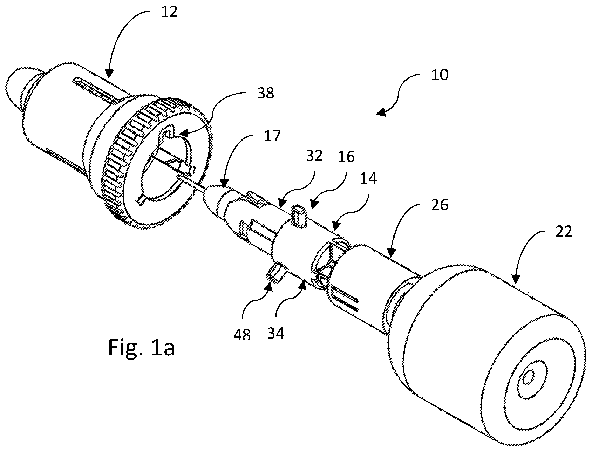

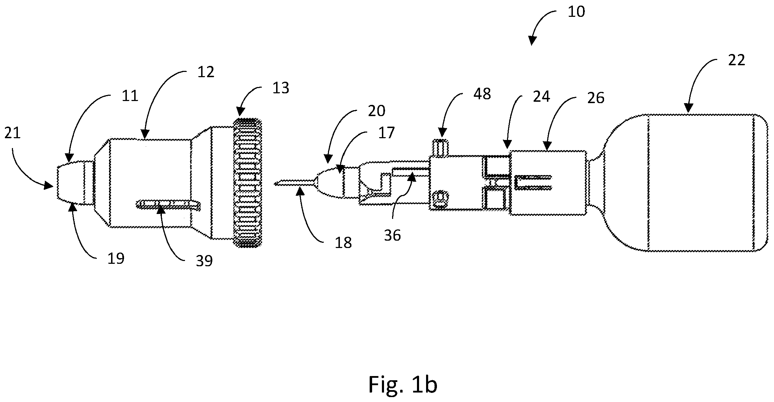

Figs 1a & 1b show exploded views of an injector 10 in accordance with an embodiment of the present invention. The injector 10 comprises an outer housing 12 having a first end 11 and second end 13. A plunger assembly 14 comprises a barrel 16 having a needle 18 disposed at a first end 20 thereof. The needle 18 is connected to the barrel 16 by way of a needle hub 17. According to the illustrated embodiment, the needle takes the form of a hypodermic needle. A fluid reservoir 22 in the form of a plastic bottle storing multiple doses of the anaesthetic is connected to a second end 24 of the barrel 16, by way of a lockable collar 26. It will be understood that in an alternative embodiment to that shown in Fig. 1, the bottle may be omitted and instead the plunger assembly 14 fluidly coupled to a draw off tube for receiving the fluid contents.

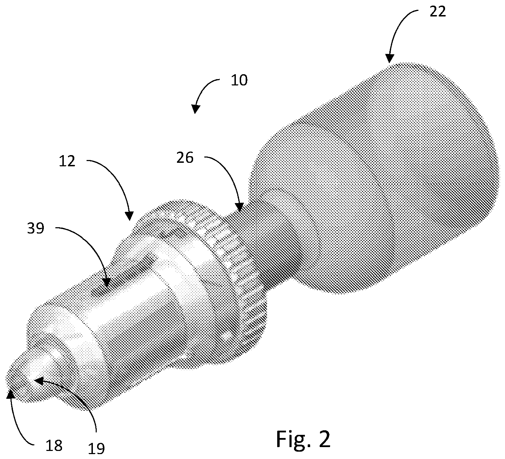

Fig. 2 shows the injector 10 in an assembled and ready for use state. The barrel 16 of the plunger assembly 14 is received within the housing 12 with the needle 18 substantially shrouded by a needle shroud 19 disposed at the first end 11 of the housing 12. In the illustrated embodiment, the needle shroud 19 is integrally formed with the housing 12. In use, the needle 18 is operable to project outwardly through a needle opening 21 in the needle shroud 19 for engaging the animal tissue. It will be understood that in an alternative embodiment, the injector 10 may be configured such that, in the ready for use state, the needle 18 is entirely shrouded by the needle shroud 19.

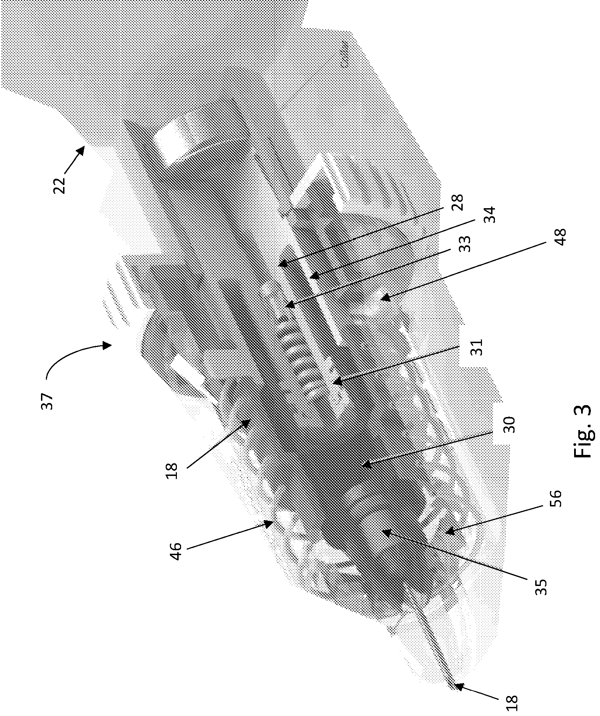

With additional reference to the sectional view of Fig. 3, the plunger assembly 14 additionally comprises a plunger 28 which is located within a chamber 30 of the barrel 16. When actuated, the plunger 28 causes fluid stored in the chamber 30 to be dispensed through the needle 18. According to the illustrated embodiment, forward movement (i.e. actuation) of the plunger 28 causes a forward portion 32 of the barrel 16 to retract into an intermediate portion 34 of the barrel 16. One or more projections (not shown) on an inner wall of the intermediate portion 34 are located within corresponding longitudinal guide channels 36 for preventing rotation of the forward portion 32 as it is retracts into the intermediate portion 34.

Also evident from Fig. 3 is a fluid channel 31 which extends through the plunger 28 and is in fluid communication with the bottle 22. A check valve 33 is located in the fluid channel 31 for permitting a one way flow of fluid from the bottle 22 into the internal chamber 30. The valve 33 may be any suitable one way valve, though according to the illustrated embodiment takes the form of a one way wire sprung check valve 35. A non-return valve 35 is located in the needle hub 17. The non-return valve 35 may be any suitable valve, including a duckbill valve, umbrella valve, radial valve or spring loaded valve.

In the assembled and ready for use state, the plunger assembly 14 is moveable between a retracted and engaged position within the housing 12. As previously stated, in the retracted position the needle 18 is partially housed within the needle shroud 19. As the plunger assembly 14 moves toward the engaged position, the needle 18 projects outwardly through the opening 21 for insertion into the animal's tissue. As will be described in more detail in subsequent paragraphs, a rotary inhibiting mechanism interacts with the plunger assembly 14 for inhibiting movement of the plunger 28 until the plunger assembly 14 has reached the engaged position (i.e. such that fluid stored within the barrel 16 can only be delivered into the tissue after the needle 18 is correctly positioned, to ensure that the full dose is delivered at the correct depth/location).

According to the illustrated embodiment, the plunger assembly 14 is moveable from the retracted to engaged position by pushing on a free end of the barrel 16, thus allowing for one handed operation. Once the plunger assembly 14 reaches the engaged position the rotary inhibiting member releases or 'unlocks' the plunger. Continued application of the pushing force causes the plunger 28 to be actuated for expelling fluid stored within the chamber 30 through the needle 18 and into the tissue. It will be understood that the force required to actuate the injector 10 is advantageously aligned with the direction of movement of the plunger assembly 14.

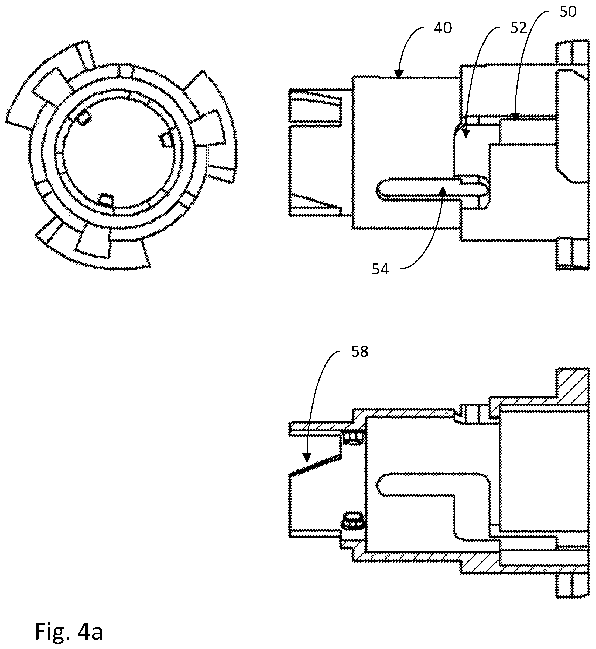

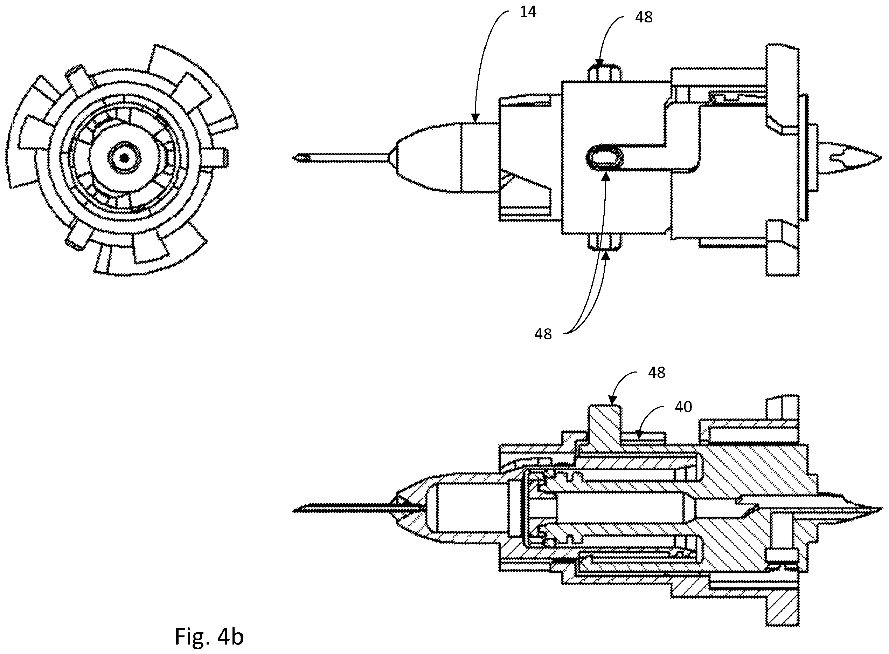

The rotary inhibiting mechanism will now be described in more detail with reference to Figs. 4a and 4b. In general terms, the mechanism comprises a rotor body 40 having a generally cylindrical and hollow body which is concentrically located within the housing 12. The rotor is shown in isolation in Fig. 4a. In the assembled state, the rotor body 40 is operable to receive the barrel 16 of the plunger assembly 14. Once received, both the rotor body 40 and plunger assembly 14 are correspondingly movable between the retracted and engaged positions (where Fig. 4b shows the rotor body and plunger mechanism in the engaged position).

Both the rotor body 40 and plunger assembly 14 are biased toward the retracted position by way of a return spring 46, as shown in Fig. 3. The return spring 46 surrounds and is co-axially aligned with the rotor body 40. The mechanism further includes at least one guide projection 48 located on the plunger assembly 14 which engages a non-seated end of the return spring 46. According to the illustrated embodiment, three guide projections 48 in the form of lugs extend outwardly from the intermediate portion 34 of the barrel 16 for engaging the return spring 46.

The barrel 16 is retained within the injector housing by way of a rotatable collar 37 located at the second end 13 of the housing 12. The rotatable collar 37 includes lug locating channels 38 defined in an inner circumferential wall. This is best shown in Fig. 1a. The locating channels 38 meet and extend into longitudinally aligned guide slots 39 in the outer housing 12 for receiving end portions of the corresponding guide projections 48. The collar 37 additionally incorporates a number of guide projections 41 (3 in this case) on the inner surface, which are provided to correctly restrict the rotor body's range angular position/movement during the different stages (assembly/in use).

At least one guide slot 50 is also defined in a circumferential wall of the rotor body 40 for receiving and guiding a corresponding guide projection 48. Since the presently described embodiment includes three projecting lugs, there are three guide slots 50 provided on the rotor body. Each guide slot 50 comprises a first transverse portion 52 (hereafter "transverse portion 52") which is in perpendicular alignment with a direction of movement of the plunger assembly 14. The transverse portion 52 extends into a longitudinal portion 54 which is in longitudinal alignment with the direction of movement.

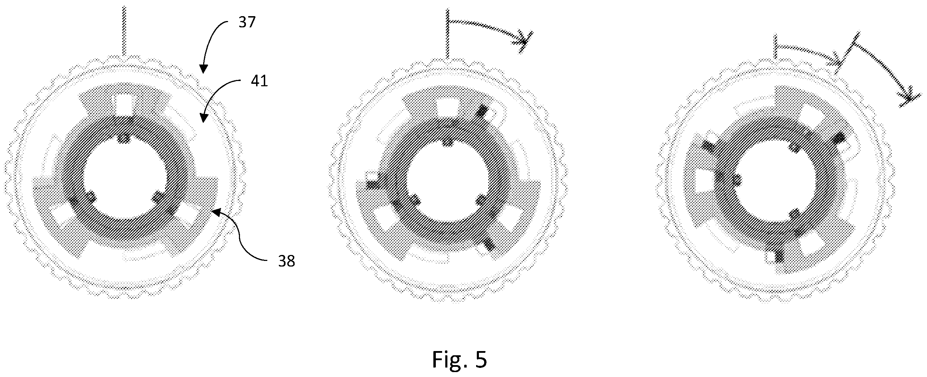

Once the barrel 16 has been received within the rotor body 40 (i.e. such that the guide projections 48 have travelled through the locating channels 38 and into respective guide slots 39, 50) the collar 37 is rotated to prevent the plunger assembly from being withdrawn from the housing 12. Fig. 5 depicts the alignment/misalignment of slots/channels in the rotatable collar 37 and the rotor body 40 during the various stages of installation. In a first stage (see the left most picture), the guide projection locating channels 38 and rotor body slots 50 are in alignment to receive the guide projections 50.

In a second stage the collar 37 is partially rotated such that the locating channels 38 and rotor body slots 50 are no longer aligned preventing the barrel 16 from escaping (see middle picture). In a third stage, the collar 37 is rotated into the locked position with the rotor body 40 rotating to the starting position.

Returning to Fig. 4, the mechanism further comprises one or more helical cams 56 disposed on an internal end wall of the housing 12 toward the first end 11. According to the illustrated embodiment there are three helical cams 56 located adjacent the needle shroud 19 and concentrically arranged around the internal wall. The helical cams 56 face correspondingly located bearing portions 58 disposed on end of the rotor body 40. As the rotor body 40 and plunger assembly 14 move toward the engaged position the helical cams 56 contact the corresponding bearing portions 58, thereby causing the rotor body 40 to rotate within the housing 12.

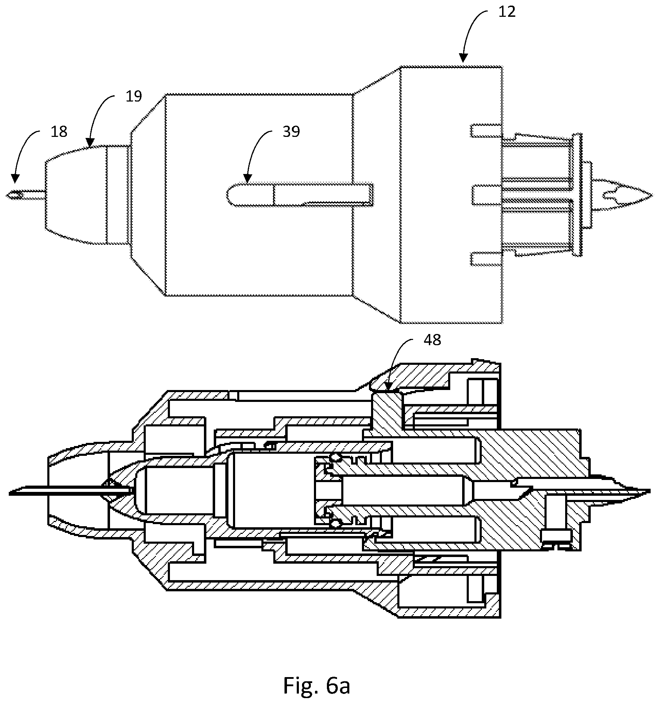

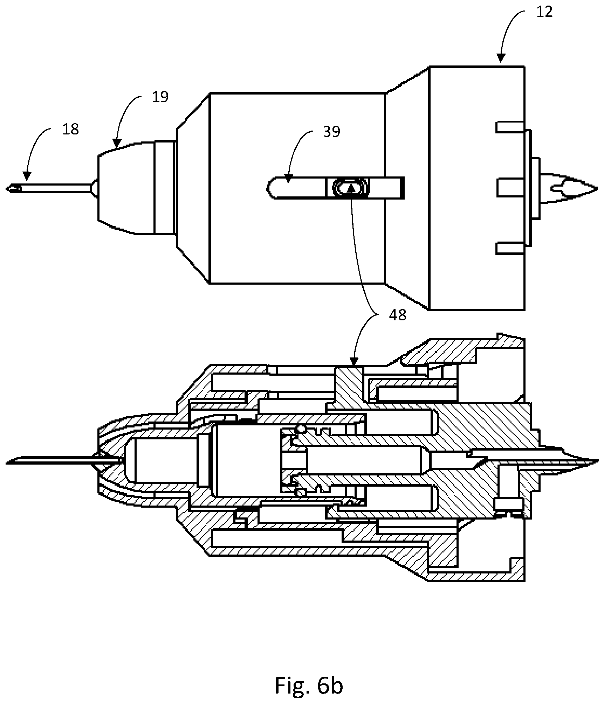

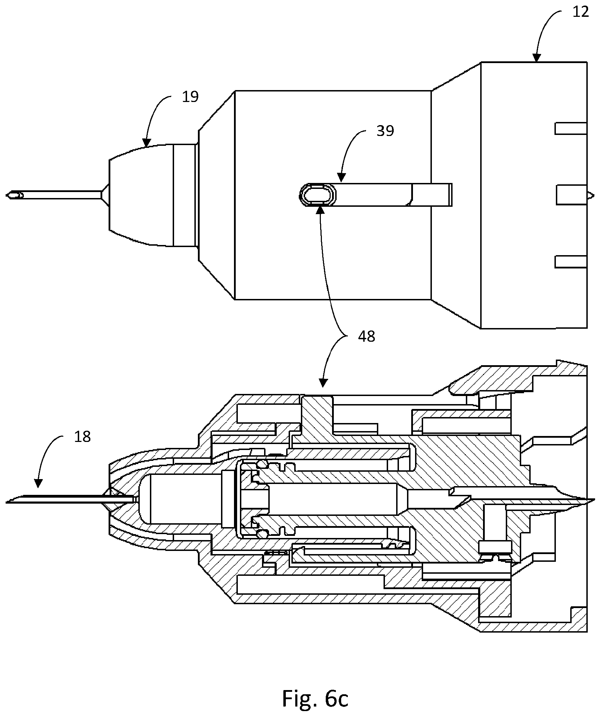

Operation of the rotary inhibiting mechanism will now be described with reference to Figs. 6a, 6b and 6c. Figure 6a shows the rotor body 40 and plunger assembly 14 in the retracted position. As shown, the rotor body 40 is aligned within the housing such that each guide projection 48 is seated on a corresponding transverse portion 52, thereby inhibiting forward movement of the plunger 28. As the bottle 22 is pushed forward, the rotor body 40 and plunger assembly 14 both move toward the engaging portion and the helical cams 56 act on the bearing portions 58 causing the rotor body 40 to turn. In Fig. 6b the rotor body 40 and plunger assembly 14 have reached the engaged state. At this point the rotor body 40 has rotated to a point where the guide projections 48 are brought into alignment with, and are free to travel down, the corresponding longitudinal portions 54. This rotational movement essentially 'unlocks' the plunger 28, thereby permitting it to be depressed through continued pushing on the bottle 22 (see Fig 6c).

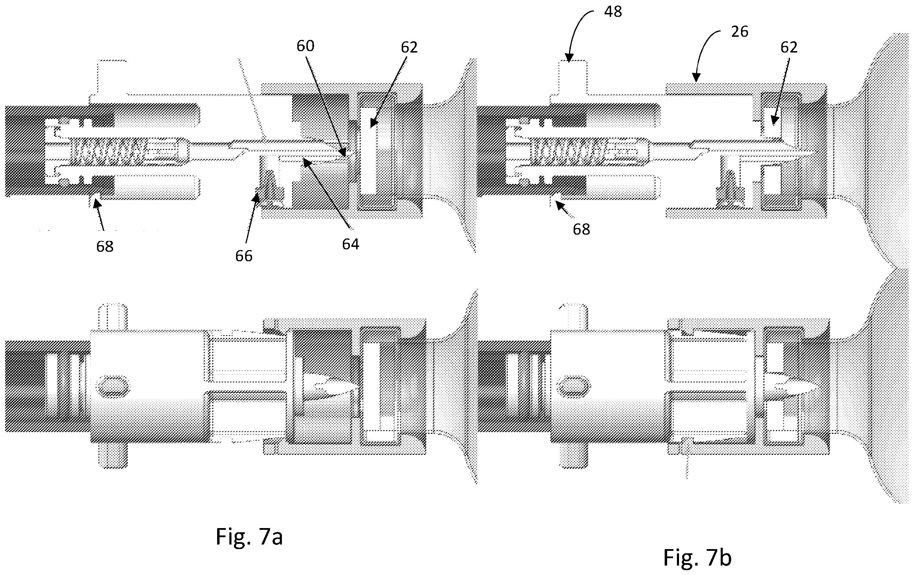

With reference to Figs. 7a and 7b there are shown, respectively, section views of the plunger assembly 14 in a partially assembled state and fully assembled "ready for use" state. As is evident from Fig. 7a, in the partially assembled state a piercing portion 60 of the intermediate barrel portion 34 is set back from an opening in the bottle 22 which is sealed by a bung 62. The fluid channel 31 extends through an opening in the piercing portion 60 (which in the illustrated embodiment takes the form of a spike). An air inlet channel 64 also extends through the opening and out a circumferential wall of the intermediate portion 34. An air inlet valve 66 is located in line with the air inlet channel 64. During insertion of the plunger assembly 14 into the housing 12 a pushing force applied to bottle 22 causes the piercing portion to move toward and broach the bung 62 thereby allowing fluid to flow into the fluid channel 31. The bottle 22 is held in the breached position by way of a snap hook fitting 68.

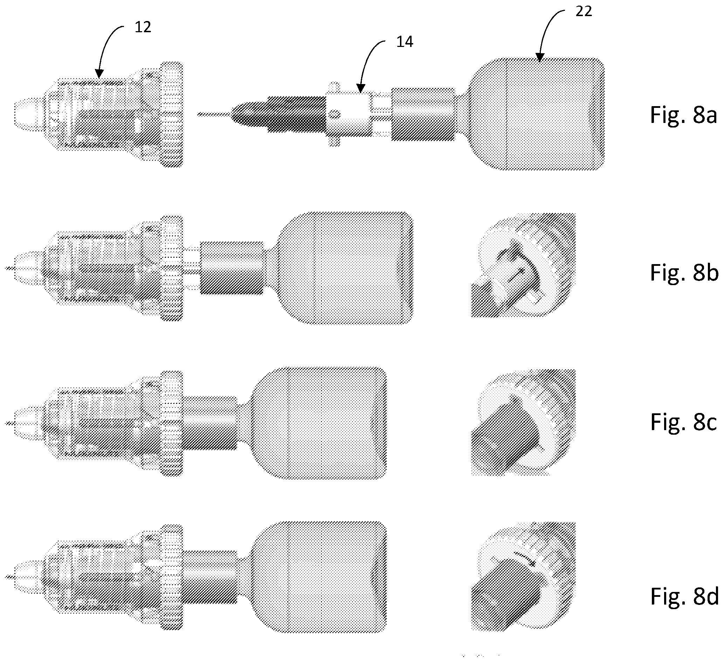

Figs. 8a to 8c illustrate steps for assembling the injector 10. Fig. 8a shows the injector is a ready to assembly position. The collar 37 of the housing 12 is in the aligned an unlocked state ready to receive the guide projections 48 of the plunger assembly 14. In Fig. 8b, the barrel 16 of the plunger assembly 14 is inserted into the housing 12, with the guide projections 48 aligned with the lug locating channels 38 in the collar 37. In Fig. 8c, the bottle 22 is pushed firmly forward, broaching the bottle 22 and pushing the plunger assembly 14 into the ready and retracted position. At this point the plunger lugs engage with the return spring. In Fig. 8c, the collar 37 is moved into the locked 'in use' position, holding the plunger assembly 14 in place and moving the rotor body 40 into the working position. The injector 10 is now ready for priming.

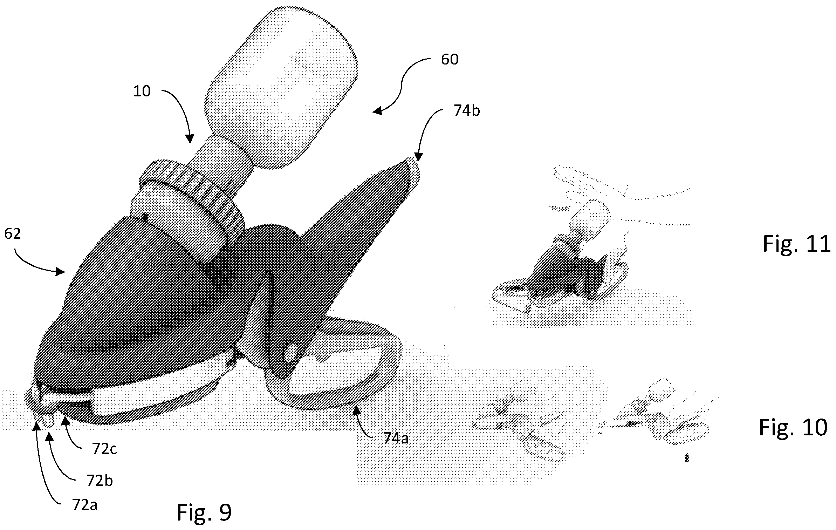

Embodiments of the present invention may also find application for delivering an anaesthetic and/or analgesic as part of a castration procedure, as will now be described with reference to Figs. 9 to 11.

Fig. 9 illustrates an apparatus 60 for removal of an animal body part. The apparatus 60 includes a ligature applicator 62 and an injector 10 as hereinbefore described. The ligature application takes much the same form as the ligature application described in UK Patent Application No. 1420805.2 (to the same applicants).

As described further below, the ligature applicator 62 is configured to move a ligature (not illustrated) between a first, extended state in which the ligature can be passed over the animal body part (not illustrated) to a selected application position, and a second, retracted state in which the ligature applies a compressive force at the application position to restrict blood flow in the animal body part. In a particular embodiment, the applicator 62 delivers the anaesthetic to the cranial side of the body part to ensure that flow to the nerves is not inhibited by the ring.

The apparatus 60 is configured to operate with a rubber/latex ring. The ligature may be a closed elastic ligature capable of being applied in a stretched condition. In the embodiment illustrated and described here the ligature applicator 60 comprises three pin members 72a, 72b, 72c. The members 72a, 72b, 72c are moveable relative to one another by operation of two handle portions 74a, 74b, as described below. The pin members 72a, 72b, 72c of the ligature applicator 62 are configured to engage with and move the ligature from an initial, retracted state to a first, extended state, as shown in Fig. 10.

As illustrated, the apparatus 60 is in the form of a hand tool. A body portion 76 detachably receives the injector 10. The injector 10 is located in the body portion 76 such that the needle opening in the injector 10 is in alignment with a portion 78 of the ligature applicator 62 which receives the animal body part. Operation of the injector 10 causes the needle to move from a retracted position whereby the needle is remote from the body part to an engaged position whereby it is engaged with the body part for delivering the anaesthetic and/or analgesic (see Fig. 11). The return spring of the injector is configured to automatically return the plunger assembly 14 of the injector 10 to the first, retracted position after the plunger 16 has been depressed.

It should be appreciated that the applicator 60 may be configured to deliver other substances to the animal. For example, the substance may be selected from one or more of the group consisting of: anaesthetics, analgesics, medicines, medicinal products, medicinal treatments, vaccines, anthelmintics, nutrients, micronutrients, minerals, vitamins, growth promotants, short acting growth promotants, tracer materials, animal identification materials, prophylactics, therapeutics, additives, insect control substances, wound dressings, bioactives, trace elements and validation markers.

The apparatus 60 is configured such that the ligature applicator 62 and the injector 10 can apply the ligature and the anaesthetic and/or analgesic substantially simultaneously. This is very convenient for the user, as it reduces the time required to work on the animal.

Reference herein to "anaesthetic" is intended to cover any substance with sensation eliminating properties and reference herein to "analgesic" is intended to cover any substance with pain-reducing or pain-relieving properties.

As noted above, although the apparatus has been described with particular reference to the castration of lambs it is also applicable to procedures for removal of other body parts. It may for example be used for the castration of other male mammals such as calves and pigs, for tail docking for example of lambs and pigs, among others.

The injector is also suitable for use with plants, e.g. injecting weeds, trees (e.g. where in the first stage the needle is inserted through the bark of the tree to a desired depth before dispensing a desired substance into the wood in the second stage), etc. The injector may also be used for impregnating plant matter, injecting telephone poles with a substance to stop them from rotting, among other uses. It will be understood that the injector described herein could be used in any application where you; move a 'fluid dispensing tip' from a known first position to a set distance second position before dispensing its contents.

In the claims which follow and in the preceding description of the invention, except where the context requires otherwise due to express language or necessary implication, the word "comprise" or variations such as "comprises" or "comprising" is used in an inclusive sense, i.e. to specify the presence of the stated features but not to preclude the presence or addition of further features in various embodiments of the invention.

## Claims

1. An injector (10) having a two-stage injection mechanism, the injector comprising:
an outer housing (12) having a first end (11) and a second end (13), the outer housing (12) comprising a cylindrical hollow body having one or more helical cams (56) disposed on an internal wall within the body at the first end;
a cylindrical hollow rotor body (40) having one or more cam bearing portions (58) disposed at one end and at least one guide slot (50) disposed in a circumferential wall, each guide slot (50) comprising a first transverse portion (52) extending into a second longitudinal portion (54), the rotary body being concentrically located within the body of the outer housing such that each cam bearing portion (58) faces a corresponding helical cam (56);
a plunger assembly (14) comprising:
a barrel (16) for storing a fluid;
a needle (18) disposed at a first end (20) of the barrel (16); and
a plunger (28) for dispensing the fluid through the needle (16); and
wherein at least one guide projection (48) is disposed on a circumferential wall of the plunger (28);
the plunger assembly (14) being at least partially locatable within the rotor body (40) such that each guide projection (48) locates in a corresponding guide slot (50) and such that when the projections (48) are located in the transverse portion (52) the plunger (28) is constrained from movement toward the first end of the barrel (16) and when located in the longitudinal portion (54) the plunger is free to move toward the first end (20) of the barrel (16);
in use the plunger assembly (14) and rotor body (40) being moveable within the outer housing (12) between a retracted position wherein the needle (18) is at least partially housed within the outer housing (12) and an engaged position wherein the needle (18) projects outwardly of a needle opening (21) located at the first end (11) of the outer housing (12),
wherein the plunger assembly (14) and rotor body (40) are moveable to the engaged position responsive to a force aligned with the direction of movement of the plunger assembly (14) being applied to a second end of the plunger assembly (14), and such that continued application of the force causes the one or more cam bearing portions (58) to bear on the one or more helical cams (56), thereby causing the rotor body (40) to rotate within the outer housing (12) in turn causing the guide projections (48) to align with longitudinal portion (54) of the corresponding guide slot (50), thereby permitting movement of the plunger for dispensing the fluid.

2. An injector (10) in accordance with claim 1, wherein the rotor body (40) and plunger assembly (14) are biased toward the retracted position by way of a return spring (46) located within the outer housing (12).

3. An injector (10) in accordance with claim 2, wherein the return spring (46) surrounds and is co-axially aligned with the rotor body (40) and wherein a portion of at least one of the rotor body and plunger assembly bears on a non-seated end of the spring.

4. An injector (10) in accordance with any one of the preceding claims, wherein the barrel (16) of the plunger assembly (14) comprises a forward portion (32) comprising a fluid chamber (30) and an intermediate portion (34) comprising a plunger head which locates within the chamber (30), and wherein a circumferential wall of the intermediate portion (34) surrounds and receives the forward portion (32) as the plunger (28) is depressed.

5. An injector (10) in accordance with claim 4, wherein, the projections (48) are lugs which extend outwardly from the circumferential wall of the intermediate portion (34).

6. An injector (10) in accordance with claim 5, wherein when in the retracted position, the rotor body (40) is aligned within the outer housing (12) such that each lug (48) seats on the transverse portion (52) of the respective guide slot (50) thereby inhibiting movement of the plunger (28).

7. An injector (10) in accordance with claim 6, wherein the outer housing (12) further comprises at least one longitudinally extending guide slot (39) disposed in the circumferential wall configured to receive an end portion of a corresponding one of the lugs (48) once the plunger assembly (14) has reached the engaged position, wherein each end portion is held within and travels along the slot (39) as the plunger assembly (14) is operated thereby preventing rotational movement of the plunger assembly (14) within the outer housing (12).

8. An injector (10) in accordance with any one of claims 5 to 7, further comprising a rotatable collar (37) located at the second end (13) of the outer housing and having locating channels (38) disposed in an inner circumferential wall thereof and wherein the collar is rotatable between an: (a) open position whereby the locating channels (38) are each axially aligned with and extend into respective guide slots (39) in the outer housing (12) for receiving a corresponding lug (48) during insertion of the plunger assembly (14) into the outer housing (12); and (b) a closed position whereby the respective slots (39) are no longer aligned, thereby preventing the plunger assembly (14) from being removed from the outer housing (12).

9. An injector (10) in accordance with any one of claims 4 to 8, wherein the plunger assembly (14) further comprising a fluid storage receptacle (22) in fluid communication with the chamber of the barrel (16), and wherein the fluid storage receptacle (22) comprises a bottle coupled to the second end (24) of the barrel (16).

10. An injector (10) in accordance with any one of claims 1 to 9, for use in delivering an anaesthetic to a body part of an animal that is to be castrated.

11. An apparatus (60) for removal of an animal body part, the apparatus comprising:
a ligature applicator (62) configured to move a ligature between a first, extended state in which the ligature can be passed over the animal body part to a selected application position, and a second, retracted state in which the ligature applies a compressive force at the application position to restrict blood flow in the animal body part; and
an injector (10) as claimed in any one of claims 1 to 9.

## Patentansprüche

1. Injektor (10) mit einem zweistufigen Injektionsmechanismus, wobei der Injektor Folgendes umfasst:
ein Außengehäuse (12) mit einem ersten Ende (11) und einem zweiten Ende (13), wobei das Außengehäuse (12) einen zylindrischen Hohlkörper mit einem oder mehreren schraubenförmigen Nocken (56) umfasst, die an einer Innenwand innerhalb des Körpers an dem ersten Ende angeordnet sind;
einen zylindrischen hohlen Rotorkörper (40) mit einem oder mehreren Nockenlagerabschnitten (58), die an einem Ende angeordnet sind, und mindestens einem Führungsschlitz (50), der in einer Umfangswand angeordnet ist, wobei jeder Führungsschlitz (50) einen ersten Querabschnitt (52) umfasst, der sich in einen zweiten Längsabschnitt (54) erstreckt, wobei der Rotorkörper konzentrisch innerhalb des Körpers des Außengehäuses angeordnet ist, so dass jeder Nockenlagerabschnitt (58) einem entsprechenden schraubenförmigen Nocken (56) gegenüberliegt;
eine Kolbenanordnung (14), die Folgendes umfasst:
ein Gefäß (16) zum Speichern eines Fluids;
eine Nadel (18), die an einem ersten Ende (20) des Gefäßes (16) angeordnet ist; und
einen Kolben (28) zum Ausgeben des Fluids durch die Nadel (16); und
wobei mindestens ein Führungsvorsprung (48) an einer Umfangswand des Kolbens (28) angeordnet ist;
wobei die Kolbenanordnung (14) sich zumindest teilweise innerhalb des Rotorkörpers (40) befinden kann, so dass jeder Führungsvorsprung (48) sich in einem entsprechenden Führungsschlitz (50) befindet, und so dass, wenn sich die Vorsprünge (48) in dem Querabschnitt (52) befinden, der Kolben (28) an einer Bewegung in Richtung des ersten Endes des Gefäßes (16) gehindert wird, und wenn sie sich in dem Längsabschnitt (54) befinden, der Kolben sich frei in Richtung des ersten Endes (20) des Gefäßes (16) bewegen kann;
wobei im Gebrauch die Kolbenanordnung (14) und der Rotorkörper (40) innerhalb des Außengehäuses (12) zwischen einer zurückgezogenen Position, in der die Nadel (18) zumindest teilweise innerhalb des Außengehäuses (12) untergebracht ist, und einer Eingriffsposition beweglich sind, in der die Nadel (18) aus einer Nadelöffnung (21) herausragt, die sich an dem ersten Ende (11) des Außengehäuses (12) befindet,
wobei die Kolbenanordnung (14) und der Rotorkörper (40) in die Eingriffsposition als Reaktion auf eine mit der Bewegungsrichtung der Kolbenanordnung (14) ausgerichtete Kraft, die auf ein zweites Ende der Kolbenanordnung (14)angewendet wird, beweglich sind, und zwar so, dass die fortgesetzte Anwendung der Kraft bewirkt, dass der eine oder die mehreren Nockenlagerabschnitte (58) auf dem einen oder den mehreren schraubenförmigen Nocken (56) aufliegen, wodurch der Rotorkörper (40) veranlasst wird, sich innerhalb des Außengehäuses (12) zu drehen, was wiederum bewirkt, dass sich die Führungsvorsprünge (48) zu dem Längsabschnitt (54) des entsprechenden Führungsschlitzes (50) ausrichten, wodurch eine Bewegung des Kolbens zur Abgabe des Fluids ermöglicht wird.

2. Injektor (10) nach Anspruch 1, wobei der Rotorkörper (40) und die Kolben-anordnung (14) durch eine sich im Außengehäuse (12) befindende Rückstellfeder (46) in Richtung der zurückgezogenen Position vorgespannt sind.

3. Injektor (10) nach Anspruch 2, wobei die Rückstellfeder (46) den Rotorkörper (40) umgibt und mit diesem koaxial ausgerichtet ist, und wobei ein Abschnitt des Rotorkörpers und/oder der Kolbenanordnung auf einem nicht gelagerten Ende der Feder aufliegt.

4. Injektor (10) nach einem der vorhergehenden Ansprüche, wobei das Gefäß (16) der Kolbenanordnung (14) einen vorderen Abschnitt (32) mit einer Fluidkammer (30) und einen Zwischenabschnitt (34) mit einem Kolbenkopf umfasst, der sich in der Kammer (30) befindet, und wobei eine Umfangswand des Zwischenabschnitts (34) den vorderen Abschnitt (32) umgibt und aufnimmt, wenn der Kolben (28) niedergedrückt ist.

5. Injektor (10) nach Anspruch 4, wobei die Vorsprünge (48) Zapfen sind, die sich von der Umfangswand des Zwischenabschnitts (34) nach außen erstrecken.

6. Injektor (10) nach Anspruch 5, wobei der Rotorkörper (40) in der zurückgezogenen Position innerhalb des Außengehäuses (12) so ausgerichtet ist, dass jeder Zapfen (48) auf dem Querabschnitt (52) des jeweiligen Führungsschlitzes (50) sitzt, wodurch die Bewegung des Kolbens (28) verhindert wird.

7. Injektor (10) nach Anspruch 6, wobei das Außengehäuse (12) ferner mindestens einen sich in Längsrichtung erstreckenden Führungsschlitz (39) umfasst, der in der Umfangswand angeordnet ist, die so konfiguriert ist, dass sie einen Endabschnitt eines entsprechenden der Zapfen (48) aufnimmt, sobald die Kolbenanordnung (14) die Eingriffsposition erreicht hat, wobei jeder Endabschnitt innerhalb des Schlitzes (39) gehalten wird und sich entlang dieses bewegt, wenn die Kolbenanordnung (14) betätigt wird, wodurch eine Rotationsbewegung der Kolbenanordnung (14) innerhalb des Außengehäuses (12) verhindert wird.

8. Injektor (10) nach einem der Ansprüche 5 bis 7, ferner umfassend einen drehbaren Kragen (37), der sich an dem zweiten Ende (13) des Außengehäuses befindet und in einer inneren Umfangswand desselben angeordnete Positionierkanäle (38) hat, wobei der Kragen drehbar ist zwischen: (a) einer offenen Position, in der die Positionierkanäle (38) jeweils axial zu entsprechenden Führungsschlitzen (39) im Außengehäuse (12) ausgerichtet sind und sich in diese hinein erstrecken, um einen entsprechenden Zapfen (48) während des Einsetzens der Kolbenanordnung (14) in das Außengehäuse (12) aufzunehmen; und (b) einer geschlossenen Position, in der die entsprechenden Schlitze (39) nicht mehr ausgerichtet sind, wodurch verhindert wird, dass die Kolbenanordnung (14) aus dem Außengehäuse (12) entfernt wird.

9. Injektor (10) nach einem der Ansprüche 4 bis 8, wobei die Kolbenanordnung (14) ferner einen Flüssigkeitsspeicherbehälter (22) mit der Kammer des Gefäßes (16) in Fluidverbindung umfasst, und wobei der Flüssigkeitsspeicherbehälter (22) eine Flasche umfasst, die mit dem zweiten Ende (24) des Gefäßes (16) verbunden ist.

10. Injektor (10) nach einem der Ansprüche 1 bis 9, zur Verwendung bei der Verabreichung eines Anästhetikums an ein Körperteil eines Tieres, das kastriert werden soll.

11. Vorrichtung (60) zum Entfernen eines Körperteils eines Tieres, wobei die Vorrichtung Folgendes umfasst:
einen Ligaturapplikator (62), der so konfiguriert ist, dass er eine Ligatur zwischen einem ersten, ausgezogenen Zustand, in dem die Ligatur über den Körperteil des Tieres zu einer ausgewählten Applikationsposition geführt werden kann, und einem zweiten, zurückgezogenen Zustand, in dem die Ligatur an der Applikationsposition eine Druckkraft anwendet, um den Blutfluss in dem Körperteil des Tieres einzuschränken, bewegt; und
einen Injektor (10) nach einem der Ansprüche 1 bis 9.

## Revendications

1. Injecteur (10) ayant un mécanisme d'injection à deux étapes, l'injecteur comprenant :
un logement extérieur (12) ayant une première extrémité (11) et une seconde extrémité (13), le logement extérieur (12) comprenant un corps creux cylindrique ayant une ou plusieurs cames hélicoïdales (56) disposées sur une paroi interne à l'intérieur du corps au niveau de la première extrémité ;
un corps de rotor (40) creux cylindrique ayant une ou plusieurs parties de support de came (58) disposées au niveau d'une extrémité et au moins une fente de guidage (50) disposée dans une paroi circonférentielle, chaque fente de guidage (50) comprenant une première partie transversale (52) pénétrant dans une seconde partie longitudinale (54), le corps rotatif étant situé de manière concentrique à l'intérieur du corps du logement extérieur de sorte que chaque partie de support de came (58) fait face à une came hélicoïdale (56) correspondante ;
un ensemble piston (14) comprenant :
un cylindre (16) pour stocker un fluide ;
une aiguille (18) disposée au niveau d'une première extrémité (20) du cylindre (16) ; et un piston (28) pour distribuer le fluide à travers l'aiguille (16) ; et
dans lequel au moins une saillie de guidage (48) est disposée sur une paroi circonférentielle du piston (28) ;
l'ensemble piston (14) pouvant être au moins partiellement situé à l'intérieur du corps de rotor (40) de sorte que chaque saillie de guidage (48) se situe dans une fente de guidage (50) correspondante et de sorte que, lorsque les saillies (48) sont situées dans la partie transversale (52), le piston (28) est empêché de se déplacer vers la première extrémité du cylindre (16) et, lorsqu'elles sont situées dans la partie longitudinale (54), le piston est libre de se déplacer vers la première extrémité (20) du cylindre (16) ;
en cours d'utilisation, l'ensemble piston (14) et le corps de rotor (40) peuvent se déplacer à l'intérieur du logement extérieur (12) entre une position rétractée, dans laquelle l'aiguille (18) est au moins partiellement logée à l'intérieur du logement extérieur (12), et une position en prise, dans laquelle l'aiguille (18) fait saillie vers l'extérieur d'une ouverture d'aiguille (21) située au niveau de la première extrémité (11) du logement extérieur (12),
dans lequel l'ensemble piston (14) et le corps de rotor (40) peuvent se déplacer vers la position en prise en réponse à une force alignée avec la direction de mouvement de l'ensemble piston (14) appliquée à une seconde extrémité de l'ensemble piston (14), et de sorte qu'une application continue de la force amène les une ou plusieurs parties de support de came (58) à supporter les une ou plusieurs cames hélicoïdales (56), amenant ainsi le corps de rotor (40) à tourner à l'intérieur du logement extérieur (12), amenant à leur tour les saillies de guidage (48) à s'aligner avec une partie longitudinale (54) de la fente de guidage (50) correspondante, permettant ainsi le mouvement du piston pour distribuer le fluide.

2. Injecteur (10) selon la revendication 1, dans lequel le corps de rotor (40) et l'ensemble piston (14) sont sollicités vers la position rétractée au moyen d'un ressort de rappel (46) situé à l'intérieur du logement extérieur (12).

3. Injecteur (10) selon la revendication 2, dans lequel le ressort de rappel (46) entoure le corps de rotor (40) et est aligné coaxialement avec celui-ci, et dans lequel une partie d'au moins un parmi le corps de rotor et l'ensemble piston supporte une extrémité non appuyée du ressort.

4. Injecteur (10) selon l'une quelconque des revendications précédentes, dans lequel le cylindre (16) de l'ensemble piston (14) comprend une partie avant (32) comprenant une chambre de fluide (30) et une partie intermédiaire (34) comprenant une tête de piston qui est située à l'intérieur de la chambre (30), et dans lequel une paroi circonférentielle de la partie intermédiaire (34) entoure et reçoit la partie avant (32) lorsque le piston (28) est enfoncé.

5. Injecteur (10) selon la revendication 4, dans lequel, les saillies (48) sont des pattes qui s'étendent vers l'extérieur à partir de la paroi circonférentielle de la partie intermédiaire (34).

6. Injecteur (10) selon la revendication 5, dans lequel, lorsqu'il est en position rétractée, le corps de rotor (40) est aligné à l'intérieur du logement extérieur (12) de sorte que chaque patte (48) s'appuie sur la partie transversale (52) de la fente de guidage (50) respective, empêchant ainsi le mouvement du piston (28).

7. Injecteur (10) selon la revendication 6, dans lequel le logement extérieur (12) comprend en outre au moins une fente de guidage (39) s'étendant longitudinalement disposée dans la paroi circonférentielle conçue pour recevoir une partie d'extrémité d'une patte correspondante parmi les pattes (48) une fois que l'ensemble piston (14) a atteint la position en prise, dans lequel chaque partie d'extrémité est maintenue à l'intérieur de la fente (39) et se déplace le long de celle-ci lorsque l'ensemble piston (14) est actionné, empêchant ainsi le mouvement de rotation de l'ensemble piston (14) à l'intérieur du logement extérieur (12).

8. Injecteur (10) selon l'une quelconque des revendications 5 à 7, comprenant en outre un collier rotatif (37) situé au niveau de la seconde extrémité (13) du logement extérieur et ayant des canaux de positionnement (38) disposés dans une paroi circonférentielle intérieure de celui-ci, et dans lequel le collier peut tourner entre : (a) une position ouverte dans laquelle les canaux de positionnement (38) sont chacun alignés axialement avec des fentes de guidage (39) respectives et s'étendent dans celles-ci dans le logement extérieur (12) pour recevoir une patte (48) correspondante pendant l'insertion de l'ensemble piston (14) dans le logement extérieur (12) ; et (b) une position fermée dans laquelle les fentes (39) respectives ne sont plus alignées, empêchant ainsi l'ensemble piston (14) d'être retiré du logement extérieur (12).

9. Injecteur (10) selon l'une quelconque des revendications 4 à 8, dans lequel l'ensemble piston (14) comprend en outre un récipient de stockage de fluide (22) en communication fluidique avec la chambre du cylindre (16), et dans lequel le récipient de stockage de fluide (22) comprend une bouteille couplée à la seconde extrémité (24) du cylindre (16).

10. Injecteur (10) selon l'une quelconque des revendications 1 à 9, destiné à être utilisé dans l'administration d'un anesthésiant à une partie du corps d'un animal qui doit être castré.

11. Appareil (60) pour le retrait d'une partie du corps d'un animal, l'appareil comprenant :
un applicateur de ligature (62) conçu pour faire passer une ligature d'un premier état étendu, dans lequel la ligature peut être passée sur la partie du corps de l'animal jusqu'à une position d'application choisie, à un second état rétracté, dans lequel la ligature applique une force de compression au niveau de la position d'application pour restreindre le flux sanguin dans la partie du corps de l'animal ; et
un injecteur (10) selon l'une quelconque des revendications 1 à 9.
